(19) Europäisches Patentamt
European Patent Office
Office européen des brevets

(11) Veröffentlichungsnummer: **0 431 402 A2**

# (12) EUROPÄISCHE PATENTANMELDUNG

(21) Anmeldenummer: **90122262.0**

(22) Anmeldetag: **22.11.90**

(51) Int. Cl.[5]: **C07C 69/78**, C09K 19/20, C09K 19/30

The title of the invention has been amended (Guidelines for Examination in the EPO, A-III, 7.3).

(30) Priorität: **05.12.89 DE 3940142**
**22.03.90 DE 4009238**

(43) Veröffentlichungstag der Anmeldung:
**12.06.91 Patentblatt 91/24**

(84) Benannte Vertragsstaaten:
**DE GB**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Kurmeier, Hans-Adolf, Dr.**
**Hinter der Schule 3a**
**W-6104 Seeheim-Jugenheim(DE)**
Erfinder: **Poetsch, Eike, Dr.**
**Am Buchwald 4**
**W-6109 Mühltal(DE)**
Erfinder: **Rieger, Dr.**
**Wacore-Tamagawagakuen**
**2834 Ootadaira, Nara-machi, Midori-ku**
**Yokohama-shi, Kanagawa Pref. 227(JP)**
Erfinder: **Hittich, Reinhard, Dr.**
**Am Kirchberg 11**
**W-6101 Modautal 1(DE)**
Erfinder: **Finkenzeller, Ulrich, Dr.**
**Waldpfad 74**
**W-6831 Plankstadt(DE)**

## (54) Di- und Trifluormethoxyverbindungen und ihre Verwendung als Komponenten flüssigkristalliner Medien.

(57) Di- und Trifluormethoxyverbindungen der Formel I

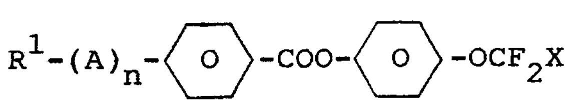

I

worin $R^1$, A und n die in Anspruch 1 angegebene Bedeutung haben, sind als Komponenten flüssigkristalliner Medien geeignet.

EP 0 431 402 A2

## DI- UND TRIFLUORMETHOXYVERBINDUNGEN

Die Erfindung betrifft Di- und Trifluormethoxyverbindungen der Formel I

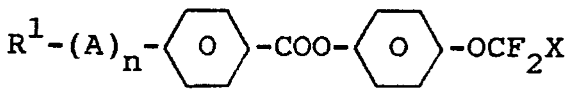 I

worin

| | |
|---|---|
| $R^1$ | Alkyl mit 1-18 C-Atomen, worin auch eine oder mehrere $CH_2$-Gruppen ersetzt sein können durch -O-, -CO- und/oder -CH=CH-, |
| X | H oder F, |
| n | 0 oder 1 |

und

A 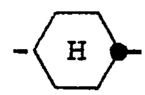, 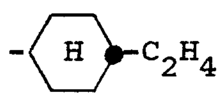 oder 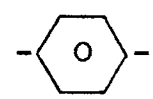

bedeutet, sowie deren Verwendung als Komponenten flüssigkristalliner Medien.

Der Einfachheit halber bedeuten im folgenden Cyc eine 1,4-Cyclohexylengruppe und Phe eine 1,4-Phenylengruppe.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedere Viskositäten bei hoher dielektrischer Anisotropie. Mit ihrer Hilfe lassen sich stabile flüssigkristalline Medien mit breitem Mesophasenbereich und vorteilhaften Werten für die optische und dielektrische Anisotropie erhalten.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zuge setzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, X, n und A die angegebene Bedeutung, sofern nicht ausdrücklich etwas anderes vermerkt ist.

Die Verbindungen der Formel I umfassen dementsprechend Verbindungen mit zwei Ringen der Teilformel Ia

$$R^1\text{-Phe-COO-Phe-OCF}_2X \qquad \text{Ia}$$

sowie Verbindungen mit drei Ringen der Teilformeln Ib bis Id

$$R^1\text{-Phe-Phe-COO-Phe-OCF}_2X \qquad \text{Ib}$$
$$R^1\text{-Cyc-Phe-COO-Phe-OCF}_2X \qquad \text{Ic}$$
$$R^1\text{-Cyc-}C_2H_4\text{-Phe-COO-Phe-OCF}_2X \qquad \text{Id.}$$

Darunter sind diejenigen der Teilformel Ia und Ic besonders bevorzugt.

In den Verbindungen der vor- und nachstehenden Formeln bedeutet $R^1$ vorzugsweise Alkyl, ferner Alkoxy. Ferner ist $R^1$ bervorzugt eine Alkylgruppe, in der eine $CH_2$-Gruppe durch -CH=CH- ersetzt ist. n ist 0 oder 1 und A Cyc, Cyc-$C_2H_4$oder Phe, vorzugsweise Cyc oder Cyc-$C_2H_4$.

Falls $R^1$ einen Alkylrest oder einen Alkoxyrest bedeutet, so kann dieser geradkettig oder verzweigt sein. Vorzugsweise ist er geradkettig, hat 2,3,4,5,6,7,8 oder 9-C-Atome und bedeutet demnach bevorzugt Ethyl, Propyl, Butyl, Pentyl, Hexyl, Heptyl, Octyl, Nonyl, Ethoxy, Propoxy, Butyloxy, Pentyloxy, Hexyloxy, Heptyloxy, Octyloxy oder Nonyloxy, ferner auch Methyl, Methoxy, Decyl, Undecyl, Dodecyl, Tridecyl, Tetradecyl, Decyloxy, Undecyloxy, Tridecyloxy oder Tetradecyloxy.

Oxaalkyl bedeutet vorzugsweise geradkettiges 2-Oxapropyl (= Methoxymethyl), 2-(=Ethoxymethyl) oder 3-Oxabutyl (= 2-Methoxyethyl), 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7-oder 8-Oxanonyl oder 2-, 3-, 4-, 5-, 6-, 7-, 8- oder 9-Oxadecyl.

Falls $R^1$ einen Alkylrest bedeutet, in dem eine $CH_2$-Gruppe durch -CH=CH-ersetzt ist, so kann dieser geradkettig oder verzweigt sein. Vorzugsweise ist er geradkettig und hat 2 bis 10 C-Atome. Er bedeutet demnach bevorzugt Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4- 5-oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formel I mit verzweigter Flügelgruppe $R^1$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verbindungen der Formel I mit $S_A$-Phasen eignen sich für thermisch adressierte Displays.

X ist vorzugsweise F.

Verzweigte Gruppen dieser Art enthalten in der Regel nicht mehr als eine Kettenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy 3-Methylbutoxy, 2-Methylpentoxy, 3-Methylpentoxy, 2-Ethylhexoxy, 1-Methylhexoxy, 2-Octyloxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Methyl-3-oxa-pentyl, 2-Methyl-3-oxahexyl.

Formel I umfaßt sowohl die Racemate dieser Verbindungen als auch die optischen Antipoden sowie deren Gemische.

Die Verbindungen der Formel I werden nach an sich bekannten Methoden dargestellt, wie sie in der Literatur (z.B. in den Standardwerken wie Houben-Weyl, Methoden der Organischen Chemie, Georg Thieme Verlag, Stuttgart Bd IX, S. 867 ff.) beschrieben sind, und zwar unter Reaktionsbedingungen, die für die genannten Umsetzungen bekannt und geeignet sind. Dabei kann man von an sich bekannten, hier nicht näher erwähnten Varianten Gebrauch machen.

Vorzugsweise werden die Ester der Formel I durch Veresterung entsprechender Carbonsäuren (oder ihren reaktionsfähigen Derivaten) mit Alkoholen bzw. Phenolen (oder ihrer reaktionfähigen Derivaten) erhalten werden. Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Na triumacetet, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bei 48 Stunden beendet.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl-oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

$$R'-L-E-R'' \quad 1$$

$$R'-L-COO-E-R'' \quad 2$$

$$R'-L-OOC-E-R'' \quad 3$$

$$R'-L-CH_2CH_2-E-R'' \quad 4$$

$$R'-L-C{\equiv}C-E-R'' \quad 5$$

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-. R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, - $CF_3$, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemaßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:
K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

Zu einem Gemisch aus 4 g 4-Trifluormethoxyphenol und 20 ml Toluol gibt man zunächst 4,6 g Dicyclohexylcarbodiimid in 80 ml Toluol, fügt dann 0,2 g Dimethylaminopyridin zu, rührt 1 Stunde bei Raumtemperatur und gibt dann 3,6 g 4-Ethylbenzoesäure zu. Nach Aufarbeitung und Reinigung durch Chromatographie und/oder Kristallisation erhält man 4-Ethylbenzoesäure-4-trifluormethoxyphenylester.

Analog werden hergestellt: 4-Methylbenzoesäure-4-trifluormethoxhphenylester
4-Propylbenzoesäure-4-trifluormethoxyphenylester, K 52° I
4-Butylbenzoesäure-4-trifluormethoxyphenylester
4-Pentylbenzoesäure-4-trifluormethoxyphenylester,
K 51° $S_A$ (32°) I
4-Hexylbenzoesäure-4-trifluormethoxyphenylester
4-Heptylbenzoesäure-4-trifluormethoxyphenylester
4-Octylbenzoesäure-4-trifluormethoxyphenylester
4-Nonylbenzoesäure-4-trifluormethoxyphenylester
4-(4-Methylphenyl)benzoesäure-4-trifluormethoxyphenylester
4-(4-Ethylphenyl)benzoesäure-4-trifluormethoxyphenylester
4-(4-Propylphenyl)benzoesäure-4-trifluormethoxyphenylester
4-(4-Butylphenyl)benzoesäure-4-trifluormethoxyphenylester
4-(4-Pentylphenyl)benzoesäure-4-trifluormethoxyphenylester
4-(4-Hexylphenyl)benzoesäure-4-trifluormethoxyphenylester

4-(4-Heptylphenyl)benzoesäure-4-trifluormethoxyphenylester
4-(4-Octylphenyl)benzoesäure-4-trifluormethoxyphenylester
4-(4-Nonylphenyl)benzoesäure-4-trifluormethoxyphenylester
4-(trans-4-Ethylcyclohexyl)benzoesäure-4-trifluormethoxyphenylester
4-(trans-4-Methylcyclohexyl)benzoesäure-4-trifluormethoxyphenylester,
4-(trans-4-Propylcyclohexyl)benzoesäure-4-trifluormethoxyphenylester, K73° $S_B$ 77° $S_A$ 126° N 173,4° I
4-(trans-4-Butylcyclohexyl)benzoesäure-4-trifluormethoxyphenylester
4-(trans-4-Pentylcyclohexyl)benzoesäure-4-trifluormethoxyphenylester, K 106° $S_B$ (84°) $S_A$ 131° N 167,9° I
4-(trans-4-Hexylcyclohexyl)benzoesäure-4-trifluormethoxyphenylester
4-(trans-4-Heptylcyclohexyl)benzoesäure-4-trifluormethoxyphenylester
4-(trans-4-Octylcyclohexyl)benzoesäure-4-trifluormethoxyphenylester
4-(trans-4-Nonylcyclohexyl)benzoesäure-4-trifluormethoxyphenylester
4-Ethoxybenzoesäure-4-trifluormethoxyphenylester
4-Propoxybenzoesäure-4-trifluormethoxyphenylester
4-Butoxybenzoesäure-4-trifluormethoxyphenylester
4-Pentyloxybenzoesäure-4-trifluormethoxyphenylester
4-Hexyloxybenzoesäure-4-trifluormethoxyphenylester
4-Heptyloxybenzoesäure-4-trifluormethoxyphenylester
4-Octyloxybenzoesäure-4-trifluormethoxyphenylester
4-(4-Ethoxyphenyl)benzoesäure-4-trifluormethoxyphenylester
4-(4-Propoxyphenyl)benzoesäure-4-trifluormethoxyphenylester
4-(4-Butoxyphenyl)benzoesäure-4-trifluormethoxyphenylester
4-(4-Pentyloxyphenyl)benzoesäure-4-trifluormethoxyphenylester
4-(4-Hexyloxyphenyl)benzoesäure-4-trifluormethoxyphenylester
4-(4-Heptyloxyphenyl)benzoesäure-4-trifluormethoxyphenylester
4-(4-Octyloxyphenyl)benzoesäure-4-trifluormethoxyphenylester
4-(trans-4-Ethylcyclohexylethyl)benzoesäure-4-trifluormethoxyphenylester
4-(trans-4-Propylcyclohexylethyl)benzoesäure-4-trifluormethoxyphenylester
4-(trans-4-Butylcyclohexylethyl)benzoesäure-4-trifluormethoxyphenylester
4-(trans-4-Pentylcyclohexylethyl)benzoesäure-4-trifluormethoxyphenylester
4-(trans-4-Hexylcyclohexylethyl)benzoesäure-4-trifluormethoxyphenylester
4-(trans-4-Heptylcyclohexylethyl)benzoesäure-4-trifluormethoxyphenylester
4-(trans-4-Octylcyclohexylethyl)benzoesäure-4-trifluormethoxyphenylester
4-(trans-4-Nonylcyclohexylethyl)benzoesäure-4-trifluormethoxyphenylester
4-Methylbenzoesäure-4-difluormethoxyphenylester
4-Ethylbenzoesaure-4-difluormethoxyphenylester
4-Propylbenzoesäure-4-difluormethoxyphenylester, K 48 I
4-Butylbenzoesaure-4-difluormethoxyphenylester
4-Pentylbenzoesäure-4-difluormethoxyphenylester
4-Hexylbenzoesäure-4-difluormethoxyphenylester
4-Heptylbenzoesäure-4-difluormethoxyphenylester
4-Octylbenzoesäure-4-difluormethoxyphenylester
4-Nonylbenzoesäure-4-difluormethoxyphenylester
4-(4-Methylphenyl)benzoesäure-4-difluormethoxyphenylester
4-(4-Ethylphenyl)benzoesäure-4-difluormethoxyphenylester
4-(4-Propylphenyl)benzoesäure-4-difluormethoxyphenylester
4-(4-Butylphenyl)benzoesäure-4-difluormethoxyphenylester
4-(4-Pentylphenyl)benzoesäure-4-difluormethoxyphenylester
4-(4-Hexylphenyl)benzoesäure-4-difluormethoxyphenylester
4-(4-Heptylphenyl)benzoesäure-4-difluormethoxyphenylester
4-(4-Octylphenyl)benzoesäure-4-difluormethoxyphenylester
4-(4-Nonylphenyl)benzoesäure-4-difluormethoxyphenylester
4-(trans-4-Ethylcyclohexyl)benzoesäure-4-difluormethoxyphenylester
4-(trans-4-Methylcyclohexyl)benzoesäure-4-difluormethoxyphenylester
4-(trans-4-Propylcyclohexyl)benzoesäure-4-difluormethoxyphenylester, K 101° N 183° I
4-(trans-4-Butylcyclohexyl)benzoesäure-4-difluormethoxyphenylester
4-(trans-4-Pentylcyclohexyl)benzoesäure-4-difluormethoxyphenylester, K 87° $S_B$ (69°) $S_A$ 96° N 177,9° I

4-(trans-4-Hexylcyclohexyl)benzoesäure-4-difluormethoxyphenylester
4-(trans-4-Heptylcyclohexyl)benzoesäure-4-difluormethoxyphenylester
4-(trans-4-Ethylcyclohexylethyl)benzoesäure-4-difluormethoxyphenylester
4-(trans-4-Propylcyclohexylethyl)benzoesäure-4-difluormethoxyphenylester
4-(trans-4-Butylcyclohexylethyl)benzoesäure-4-difluormethoxyphenylester
4-(trans-4-Pentylcyclohexylethyl)benzoesäure-4-difluormethoxyphenylester
4-(trans-4-Hexylcyclohexylethyl)benzoesäure-4-difluormethoxyphenylester
4-(trans-4-Heptylcyclohexylethyl)benzoesäure-4-difluormethoxyphenylester

Folgende Beispiele betreffen erfindungsgemäße flüssigkristalline Medien:

Beispiel A:

Ein flüssigkristallines Medium, bestehend aus 10 % 4-Pentylbenzoesäure-4-trifluormethoxyphenylester und
90 % einer Mischung, bestehend aus

24 % p-trans-4-Propylcyclohexyl-benzonitril
36 % p-trans-4-Pentylcyclohexyl-benzonitril,
25 % p-trans-4-Heptylcyclohexyl-benzonitril und

15 % 4-Cyan-4'-(trans-4-pentylcyclohexyl)-biphenyl
hat einen Klärpunkt von 61,7° und eine dielektrische Anisotropie $\Delta\epsilon$ von 13,5.

Beispiel B

Ein flüssigkristallines Medium, bestehend aus 10 % 4-Propylbenzoesäure-4-trifluormethoxyphenylester und
90 % eine Mischung, bestehend aus

24 % p-trans-4-Propylcyclohexyl-benzonitril
36 % p-trans-4-Pentylcyclohexyl-benzonitril, 25 % p-trans-4-Heptylcyclohexyl-benzonitril
und
15 % 4-Cyan-4'-(trans-4-pentylcyclohexyl)-biphenyl
hat einen Klärpunkt von 61,30 und $\Delta\epsilon$ = 13,7.

Beispiel C

Ein flüssigkristallines Medium, bestehend aus
10 % 4-(trans-4-Propylcyclohexyl)benzoesäure-4-trifluormethoxyphenylester
und
90 % eine Mischung bestehend aus

24% p-trans-4-Propylcyclohexyl-benzonitril
36 % p-trans-4-Pentylcyclohexyl-benzonitril,
25 % p-trans-4-Heptylcyclohexyl-benzonitril
und
15 % 4-Cyan-4'-(trans-4-pentylcyclohexyl)-biphenyl
hat einen Klärpunkt von 75,1° und eine dielektrische Anisotropie $\Delta\epsilon$ von 13,47.

Beispiel D

Ein flüssigkristallines Medium, bestehend aus
4% 4-(trans-4-Pentylcyclohexyl)benzoesäure-4-trifluormethoxyphenylester
und
95 % eine Mischung, bestehend aus

24 % p-trans-4-Propylcyclohexyl-benzonitril

36 % p-trans-4-Pentylcyclohexyl-benzonitril,
25 % p-trans-4-Heptylcyclohexyl-benzonitril
und
15 % 4-Cyan-4'-(trans-4-pentylcyclohexyl)-biphenyl
hat einen Klärpunkt von 72,9° und $\Delta\epsilon$ = 13,24.

### Ansprüche

1. Di- und Trifluormethoxyverbindungen der Formel I

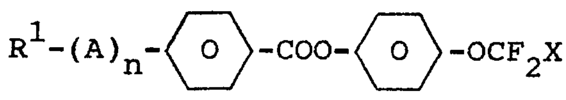

I

worin

| | |
|---|---|
| $R^1$ | Alkyl mit 1-18 C-Atomen, worin auch eine oder mehrere $CH_2$-Gruppen ersetzt sein können durch -0-, -CO- und/oder -CH=CH-, |
| X | H oder F, |
| n | 0 oder 1 |

und

A 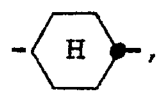, 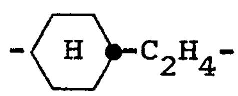 oder 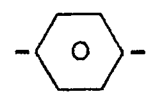

bedeutet.

2. Verwendung der Verbindungen der Formel I nach Anspruch 1 als Komponenten flüssigkristalliner Medien.

3. Flüssigkristallines Medium mit mindestens zwei flüssigkristallinen Komponenten, dadurch gekennzeichnet, daß es mindestens eine Verbindung der Formel I nach Anspruch 1 enthält.

4. Flüssigkristall-Anzeigeelement, dadurch gekennzeichnet, daß es ein flüssigkristallines Medium nach Anspruch 3 enthält.

5. Elektrooptisches Anzeigeelement, dadurch gekennzeichnet, daß es als Dielektrikum ein flüssigkristallines Medium nach Anspruch 3 enthält.